# EUROPEAN PATENT APPLICATION

(11) **EP 3 343 414 A1**
(43) Date of publication of application: **04.07.2018**
(21) Application number: 16207633.5
(22) Date of filing: 30.12.2016
(51) Int. Cl.: G06F 19/00

(54) **CONNECTION UNIT FOR CONNECTING A PLURALITY OF MEDICAL DEVICES AND SYSTEM COMPRISING SAME**

(71) Applicant: Televic Healthcare NV, 8870 Izegem (BE)
(72) Inventor: The designation of the inventor has not yet been filed
(74) Representative: IPLodge bvba

(57) **Abstract**

The invention pertains to a connection unit for connecting a plurality of medical devices to a nurse call network, the connection unit comprising: a plurality of medical device interfaces, adapted to receive respective cables connecting to respective ones of said plurality of medical devices; a network interface, adapted to connect to said nurse call network; a control interface; and electronic circuitry, operatively connected to said plurality of medical device interfaces, said network interface, and said control interface, said electronic circuitry being configured to: monitor receipt of device alarm signals and losses of connectivity at said plurality of medical device interfaces; relay received device alarm signals and detected losses of connectivity to said nurse call network over said network interface; and selectively activate and deactivate said monitoring and said relaying upon receipt of predetermined control signals over said control interface; wherein said electronic circuitry is implemented as a single non-programmable electronic device.

## Description

### Field of the Invention

The present invention pertains to the field of patient monitoring equipment, and in particular to the field of connection units for patient monitoring devices.

### Background

In hospitals, patients may be monitored by one or more medical devices that may be configured to sound an alarm when a certain vital parameter, observed by means of a sensor of the medical device, crosses a predetermined threshold, or when certain anomalies occur in the monitoring equipment (such as a cable being disconnected, or network connection being lost). The resulting alarm signals must be relayed to the relevant medical personnel, to ensure that adequate action is taken in a timely manner. Today, the relaying of such signals relies on dedicated networks, all components of which must be certified as medical devices according to applicable standards and regulations.

### Summary of the invention

According to an aspect of the present invention, there is provided a connection unit for connecting a plurality of medical devices to a nurse call network, the connection unit comprising: a plurality of medical device interfaces, adapted to receive respective cables connecting to respective ones of said plurality of medical devices; a network interface, adapted to connect to said nurse call network; a control interface; and electronic circuitry, operatively connected to said plurality of medical device interfaces, said network interface, and said control interface, said electronic circuitry being configured to: monitor receipt of device alarm signals and losses of connectivity at said plurality of medical device interfaces; relay received device alarm signals and detected losses of connectivity to said nurse call network over said network interface; and selectively activate and deactivate said monitoring and said relaying upon receipt of predetermined control signals over said control interface; wherein said electronic circuitry is implemented as a single non-programmable electronic device.

The term "single non-programmable electronic device" is used herein to describe an electronic component that does not provide the aforementioned functions by storing a user-definable list of instructions (i.e., a program) and executing these instructions, but relies on the operation of a fixed network of logic gates. Thus, the term "single non-programmable electronic device" includes for example ASICs and FPGAs that are not configured to act upon lists of instructions, and excludes microcontrollers and microprocessors that require software to perform any meaningful task.

It is an advantage of the present invention that reliable networked monitoring of medical devices can be provided over pre-existing nurse call networking infrastructure without a need for certification of new software.

In an embodiment, the connection unit according to the present invention further comprises an indicator adapted to signal an alarm condition, said electronic circuitry is further operatively connected to said indicator, and said electronic circuitry is further configured to control said indicator to signal said alarm condition upon said receipt of device alarm signals and losses of connectivity. In a particular embodiment, the indicator is adapted to signal said alarm by visual and/or auditory means.

It is an advantage of this embodiment that alarms can be notified to the locally present personnel, independently of and in addition to the relaying of the alarms over the network.

In an embodiment of the connection unit according to the present invention, said electronic circuitry is further configured to monitor a loss of connectivity at said network interface, and to activate a local alarm status when said loss of connectivity at said network interface is detected. In a particular embodiment, the connection unit further comprises a room lamp interface, and said electronic circuitry is further configured to send an alarm light signal to said room lamp interface when said local alarm status is activated.

It is an advantage of this embodiment that the cluster of monitored medical devices can be operated in a "local mode" when the network is not available. In addition, by triggering a visual alarm on the room lamp, personnel outside the patient room is immediately made aware of the loss of network connectivity and can take appropriate corrective action.

In an embodiment, the connection unit according to the present invention further comprises an authentication unit interface configured for receiving authentication unit signals from an authentication unit and providing said authentication unit signals as control signals to said electronic circuitry. In a particular embodiment, said authentication unit signals and said control signals comprise medical device port selection information. In a particular embodiment, said medical device port selection information comprises, for one or more of said plurality of medical device ports, one of a group of an activation command, a delayed activation command, and a deactivation command.

It is an advantage of this embodiment that critical operations such as activating and deactivating a medical device interface can be reserved to specific authorized persons, who must be authenticated prior to be allowed to trigger control signals.

According to an aspect of the present invention, there is provided a system comprising a connection unit as described above and at least one medical device connected to one of said medical device interfaces.

In an embodiment, the system according to the present invention further comprises an authentication unit operatively connected to said authentication unit interface.

### Brief Description of the Figures

These and other technical features and advantages of embodiments of the present invention will now be described with reference to the accompanying figure, which schematically illustrates a connection unit according to an embodiment of the present invention.

### Description of Embodiments

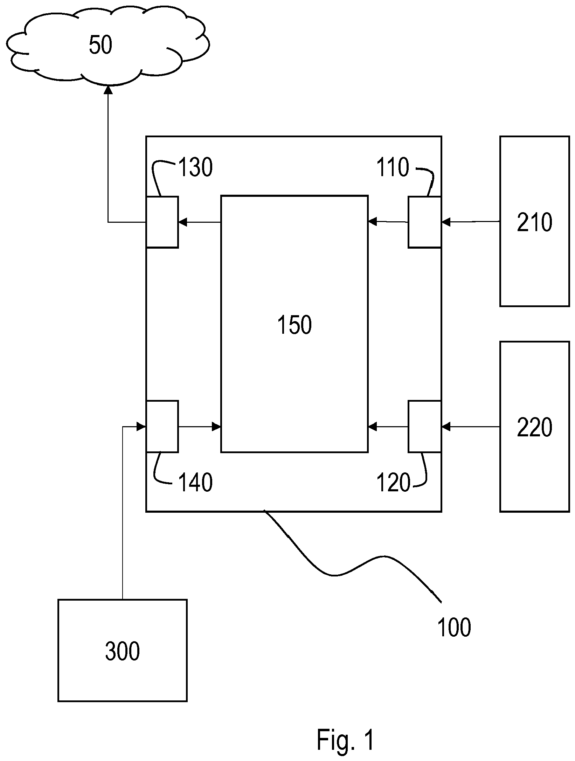

Figure 1 schematically illustrates a connection unit **100** for connecting a plurality of medical devices to a nurse call network, according to an embodiment of the present invention.

The connection unit **100** comprises a plurality of medical device interfaces to receive cables connecting to different medical devices. Without loss of generality, only two medical device interfaces **110, 120** are shown in Figure 1, having two respective medical devices **210, 220** connected to them, by means of appropriate cables and connectors (not shown in detail). The connection unit **100** may be designed in a modular way, whereby each module has a fixed number of medical device interfaces (e.g., two), and multiple modules may be interconnected to obtain a combined connection unit **100** having a larger total number of medical device interfaces. The connection unit **100** is preferably provided with status indicators (such as color-coded LEDs) to visually indicate the unit's status, which may include "on" (active), "off" (inactive), "stand-by", "error/fault", and the like (not shown in the schematic illustration). The individual medical device interfaces or ports may also be provided with status indicators (such as color-coded LEDs) to visually indicate the port's status, which may include "inactive", "active - no connection", "active - connected", "active - alarm", and the like (not shown in the schematic illustration).

The medical device interfaces **110, 120** allow the connection unit **100** to receive data (in particular medical sensor data or information derived from medical sensor data) from the attached medical devices **210, 220,** and may further allow the connection unit **100** to transmit data (in particular control data and commands affecting the operation of the devices) to the attached medical devices **210, 220.**

The connection unit **100** further comprises a network interface **130,** adapted to connect to a nurse call network **50.** A nurse call network is a network suitable for connecting patient room terminals to a central monitoring station, thus allowing patients to call for assistance when necessary. It is an advantage of the present invention that the same network, which may already be present in the premises, can be used for relaying signals from medical devices **210, 220** to the monitoring station.

The connection unit **100** further comprises a control interface, as will be described in more detail below.

The connection unit **100** further comprises electronic circuitry **150,** operatively connected to said plurality of medical device interfaces **110, 120,** said network interface **130,** and said control interface. The electronic circuitry **150** is implemented as a single non-programmable electronic device.

The electronic circuitry **150** is configured to monitor receipt of device alarm signals and losses of connectivity at the medical device interfaces **110, 120.** In order to allow detection of losses of connectivity, the medical devices **210, 220** must either send a permanent or intermittent keep-alive or "heartbeat" signal, or provide an electronic loopback of a test signal emitted by the medical device interfaces **110, 120.**

The electronic circuitry **150** is further configured to relay any received device alarm signals and any detected losses of connectivity to the nurse call network **50** over the network interface **130,** such that appropriate action can be taken by the staff that monitors the incoming alarms. The monitoring and the relaying may be selectively activated and deactivated upon receipt of predetermined control signals over the control interface **140.** In this way, individual device interfaces **110,** 120 may be turned on or off by means of appropriate control signals - this is useful to deactivate a port (device interface) that is not connected to an active device, and that might otherwise generate a permanent "loss of signal" alarm.

The connection unit **100** may further comprise an indicator adapted to signal an alarm condition, for example by visual and/or auditory means. This is useful when the alarm must be notified to staff present in the same room with the connection unit **100,** in addition to or alternatively to its being relayed over the nurse call network **50.** To this end, the electronic circuitry **150** is further operatively connected to the indicator, and the electronic circuitry 150 is further configured to control the indicator to signal the alarm condition upon receipt of device alarm signals and losses of connectivity.

The electronic circuitry **150** may be further configured to monitor a loss of connectivity at the network interface **130,** and to activate a local alarm status when loss of connectivity at the network interface 130 is detected. The local alarm status, which may be signaled by visual and/or auditory effects, provides a way to attract attention to the fact that the network connection is lost (which implies that relaying alarm signals to a centralized monitoring station is not possible). Preferably, the connection unit 100 also comprises a room lamp interface, and the connection unit 100 is further configured to send an alarm light signal to the room lamp interface when the local alarm status is activated. A room lamp or overdoor lamp is typically provided outside a patient room (e.g., above the door opening, in the hallway or corridor) to visually signal the presence of a call or an alarm situation to staff present outside the patient rooms. A room lamp may be capable of signaling various different states, by using different colors of light (or combinations of colors), flashing patterns, etc. The room lamp interface is preferably adapted to provide the necessary control signals to the room lamp to operate in accordance with various different states.

The connection unit **100** may further comprise an authentication unit interface configured for receiving authentication unit signals from an authentication unit **300** and providing these authentication unit signals as control signals to the electronic circuitry **150.** Thus, the authentication unit interface may be an external interface which connects to an internal control interface **140.** The authentication unit interface may also comprise the control interface **140,** in the sense that it may be configured to relay both control signals and other types of signals. The authentication unit signals and the control signals may include any signals that control the operation of the connection unit **100,** such as signals to activate or deactivate the connection unit **100** as a whole. The authentication unit signals and the control signals may also comprise medical device port selection information, such as, for one or more of said plurality of medical device ports, one of a group of an activation command, a delayed activation command, and a deactivation command. The deactivation command allows one of the medical device interface to be turned off, specifically when no active medical device is connected to it, while the rest of the connection unit **100** remains operational. The delayed activation command allows a medical device to be connected to a medical device interface without relaying any alarms for a predetermined time interval - the time interval may be in the order of one minute and may be used to locally generate a test alarm on the connected medical device without causing the alarm to be relayed and emergency personnel to be called in.

The authentication unit **100** may be provided with means to authenticate authorized personnel, prior to transmitting control signals to the electronic circuitry **150.** In the context of medical device port selection information, this avoids the risk of having patients or other non-authorized persons deactivate the monitoring of certain medical devices that gather critical patient data. The means to authenticate may include a keypad for receiving a PIN or a password, a biometric sensor such as a fingerprint scanner, an iris scanner, or a voice recognition device, a chip card reader, an RFID reader, a magnetic induction transceiver, and the like.

The connection unit **100** may be part of a system, along with at least one medical device **210, 220** connected to one of its medical device interfaces **110, 120,** and optionally with an authentication unit **300** operatively connected to the authentication unit interface.

While the invention has been described hereinabove with reference to specific embodiments, this was done to illustrate and not to limit the invention, the scope of which is to be determined by reference to the attached claims.

## Claims

1. A connection unit (100) for connecting a plurality of medical devices to a nurse call network, the connection unit comprising:
- a plurality of medical device interfaces (110, 120), adapted to receive respective cables connecting to respective ones of said plurality of medical devices (210, 220);
- a network interface (130), adapted to connect to said nurse call network;
- a control interface (140); and
- electronic circuitry (150), operatively connected to said plurality of medical device interfaces (110, 120), said network interface (130), and said control interface, said electronic circuitry (150) being configured to:
o monitor receipt of device alarm signals and losses of connectivity at said plurality of medical device interfaces (110, 120);
o relay received device alarm signals and detected losses of connectivity to said nurse call network over said network interface (130); and
o selectively activate and deactivate said monitoring and said relaying upon receipt of predetermined control signals over said control interface;
wherein said electronic circuitry (150) is implemented as a single non-programmable electronic device.

2. The connection unit (100) according to claim 1, further comprising an indicator adapted to signal an alarm condition, wherein said electronic circuitry (150) is further operatively connected to said indicator, and wherein said electronic circuitry (150) is further configured to control said indicator to signal said alarm condition upon said receipt of device alarm signals and losses of connectivity.

3. The connection unit (100) according to claim 2, wherein said indicator is adapted to signal said alarm by visual and/or auditory means.

4. The connection unit (100) according to any of the preceding claims, wherein said electronic circuitry (150) is further configured to monitor a loss of connectivity at said network interface (130), and to activate a local alarm status when said loss of connectivity at said network interface (130) is detected.

5. The connection unit (100) according to claim 4, further comprising a room lamp interface, wherein said electronic circuitry (150) is further configured to send an alarm light signal to said room lamp interface when said local alarm status is activated.

6. The connection unit (100) according to claim 1, further comprising an authentication unit interface configured for receiving authentication unit signals from an authentication unit (300) and providing said authentication unit signals as control signals to said electronic circuitry (150).

7. The connection unit (100) according to claim 6, wherein said authentication unit signals and said control signals comprise medical device port selection information.

8. The connection unit (100) according to claim 7, wherein said medical device port selection information comprises, for one or more of said plurality of medical device ports, one of a group of an activation command, a delayed activation command, and a deactivation command.

9. A system comprising a connection unit (100) according to any of the preceding claims and at least one medical device (210, 220) connected to one of said medical device interfaces (110, 120).

10. The system according to claim 9, further comprising an authentication unit (300) operatively connected to said authentication unit interface.
